# DEMANDE DE BREVET EUROPEEN

(11) **EP 0 953 649 A1**
(43) Date de publication de la demande: **03.11.1999**
(21) Numéro de dépôt: 99420083.0
(22) Date de dépôt: 01.04.1999
(51) Int. Cl.: C12N 15/87, C12N 15/82, C12N 15/89, C08G 77/388, C08G 83/00

(54) **Silicone azotée utile pour compacter les séquences d'acides nucléiques et utilisation pour la transfection de cellules**

(30) Priorité: 06.04.1998 FR 9804510
(71) Demandeur: Rhone Poulenc Agro, 69009 Lyon (FR)
(72) Inventeur: Decor, Rachel, 67000 Strasbourg (FR); Mioskowski, Charles, 67200 Strasbourg (FR); Wagner, Alain, 67100 Strasbourg (FR); Schmutz, Marc, 67730 Dingsheim (FR)
(74) Mandataire: Tetaz, Franck

(57) **Abrégé**

La présente invention concerne un procédé de transformation de cellules par lequel on introduit dans les cellules une quantité appropriée de fragments d'acides nucléiques. Les fragments d'acides nucléiques sont introduits sous la forme d'une composition d'acide nucléique comprenant une silicone azotée, utile pour compacter les fragments d'acides nucléiques, les compositions comprenant les agrégats de fragments d'acides nucléiques et des silicones selon l'invention.

## Description

La présente invention concerne un procédé de transformation de cellules par lequel on introduit dans les cellules une quantité appropriée de fragments d'acides nucléiques. Les fragments d'acides nucléiques sont introduits sous la forme d'une composition d'acide nucléique qui comprend dans un véhicule approprié au moins un fragment d'acide nucléique et au moins un agent compactant comprenant une silicone azotée, utile pour compacter les fragments d'acides nucléiques, les compositions comprennent les agrégats de fragments d'acides nucléiques et des silicones selon l'invention.

Pour transformer les cellules par génie génétique en introduisant un fragment d'acide nucléique étranger à ladite cellule (ou hétérologue) par transfection, il est nécéssaire au préalable de compacter les fragments d'acides nucléiques. Ce compactage se fait au moyen de composés polyaminés hyperbranchés comme la spermine ou les polyéthylèneimines (Garcia Ramirez & al., Biopolymers, vol. 34, 1984, pp. 285-292 ; WO 97 06833). Il est également connu d'utiliser des monomères de silanes pour condenser l'ADN en vue de son étude structurale sans toutefois chercher à transformer les cellules (FANG Y AND HOH J: « Surface-directed DNA condensation in the absence of soluble multivalent cations » *Nucleic acids Research,* vol. 26, n°2, 15 janvier 1998, pages 588-593.).

Il est toutefois nécessaire de trouver de nouveaux agents compactants de manière à obtenir une bonne protection des acides nucléiques, et permettre de varier les milieux utiles pour la transfection des cellules selon les applications. La présente invention apporte une solution à ce problème.

La présente invention concerne un procédé de transformation de cellules par lequel on introduit dans les cellules une quantité appropriée de fragments d'acides nucléiques, caractérisé en ce que les fragments d'acides nucléiques sont introduits sous la forme d'une composition d'acide nucléique qui comprend dans un véhicule approprié au moins un fragment d'acide nucléique et au moins un agent compactant comprenant une silicone azotée.

Selon la présente invention, on entend par « fragment d'acide nucléique » une séquence nucléotidique pouvant être de type ADN ou ARN, de préférence de type ADN, notamment double brin.

Par silicone azotée on entend selon l'invention tout composé comprenant dans sa structure au moins un atome de silicium sur lequel est greffé au moins un radical hydrocarboné azoté comprenant au moins un atome d'azote.

La structure de la silicone azotée selon l'invention est un oligomère. On entend par oligomère un enchaînement de monomères d'au moins 2 silanes et moins de 30 silanes, avantageusement comprenant 3 à 20 silanes.

L'atome d'azote dans le radical hydrocarboné selon l'invention se trouve sous la forme d'une amine primaire, secondaire ou tertiaire, de préférence primaire, éventuellement sous la forme d'un sel d'ammonium, ou encore sous la forme de fonctions de type guanidine ou amidine.

De manière avantageuse, le radical hydrocarboné azoté selon l'invention est un radical hydrocarboné aminé, comprenant au moins une fonction amine telle que définie ci-dessus, de préférence au moins une fonction amine primaire (-NH2).

Par radical hydrocarboné, on entend selon l'invention tout radical constitué essentiellement par des atomes de carbone et d'hydrogène, les hydrogènes étant éventuellement remplacés en partie ou totalement par des halogènes, et comprenant éventuellement des atomes d'oxygène, d'azote, de soufre ou de phosphore.

De manière préférentielle, le radical hydrocarboné est choisi parmi les radicaux alkyle, cycloalkyle, aryle et les radicaux polycycliques comprenant plusieurs cycles aliphatiques et/ou aromatiques.

Par radical alkyle, on entend de préférence selon l'invention tout radical alkyle, linéaire ou ramifié, saturé ou insaturé, éventuellement interrompu par un ou plusieurs atomes d'oxygène, et dans le cas de plusieurs atomes d'oxygène ces derniers ne sont pas adjacents, éventuellement interrompu par une ou plusieurs fonctions amine secondaire ou tertiaire, carbonyle, carbonyloxy ou oxycarbonyle, carbonate, carbamate, urée ou analogues, éventuellement substitué par un ou plusieurs groupes choisis parmi les halogènes, hydroxy ou amine primaires, secondaire ou tertiaire, ou radicaux cycloalkyle, aryle ou polycycliques. De manière avantageuse, le radical alkyle selon l'invention comprend de 1 à 10 atomes de carbone, de préférence au moins trois atomes de carbone, plus préférentiellement de 3 à 5 atomes de carbone. Avantageusement, le radical alkyle est choisi parmi les radicaux méthyle, éthyle, propyle, isopropyle, butyle, isobutyle, tertiobutyle, pentyle et les différents isomères du pentyle.

Cette définition des radicaux alkyles s'applique également aux radicaux cycloalkyles selon l'invention, étant entendu que les cycles comprennent au moins trois éléments, comprenant notamment les radicaux cyclopropyle, cyclopentyle, cyclobutyle ou cyclohexyle, de même que les oses, notamment pentoses ou hexoses tels que les dérivés de glucose.

Par radical aryle, on entend selon l'invention tout radical aryle ou hétéroaryle, éventuellement substitué par un ou plusieurs radicaux choisis parmi les radicaux alkyle cycloalkyle, ou polycycliques et/ou par un ou plusieurs groupes choisis parmi les halogènes, hydroxy ou amine primaire, secondaire ou tertiaire.

De manière préférentielle, le radical hydrocarboné aminé selon l'invention, est choisi parmi les radicaux 3-aminopropyle ou 3-(2-aminoéthylamino)-propyle.

Parmi les silicones aminées selon l'invention, on trouve les silicones organiques ou les dérivés de silice.

Les silicones organiques selon l'invention comprennent au moins un motif silicone suivant :

Si R1 O_{(3-x-y)/2} R2ₓ R3_{y}

dans lequel,
R1 est un radical hydrocarboné azoté, tel que défini ci-dessus,
R2 représente le radical R1, un radical hydroxyle ou un radical hydrocarboné,
R3 représente R2 ou le radical de formule 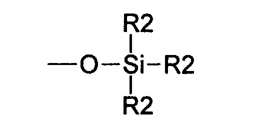
dans laquelle R2 est défini ci-dessus,
x et y, identiques ou différents sont 0, 1 ou 2, étant entendu que la somme x+y est inférieure ou égale à 3.

Selon un mode particulier de réalisation de l'invention, la silicone aminée est choisie parmi les silicones de formule générale I ou II suivantes : 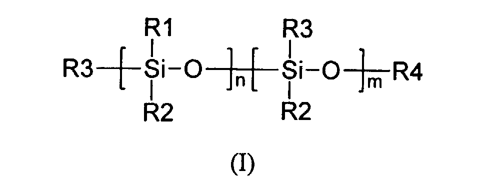 ou 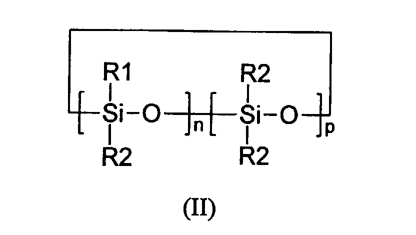 dans lesquelles,
R1, R2 et R3 sont définis ci-dessus,
R4 représente un atome d'hydrogène, un radical hydrocarboné ou un radical de formule 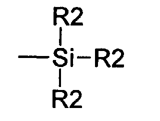
dans laquelle R2 est défini précédemment,
n est au moins égal à 1, m est supérieur ou égal à 0 et p est supérieur ou égal à 0, à la condition que la somme n+p soit supérieure ou égale à 3.

De manière préférentielle, n est compris entre 1 et 10, plus préférentiellement entre 5 et 9, et m ou p sont compris entre 0 et 9.

Selon un mode particulier de réalisation de l'invention, dans les silicones de formule générale I, R2 représente un radical hydroxyle ou un radical hydrocarboné, en particulier alkyle, R3 représente un radical hydroxyle et R4 représentent un atome d'hydrogène, n est compris entre 7 et 9 et m est égal à 0.

Les silicones selon l'invention sont préparées selon les méthodes usuelles de la techniques, décrites notamment dans *Organic Chemistry* (vol. 29, Polymer synthese, vol. I, chap. 4, pp. 114-139), par *Patai & Rappopor* (The Chemistry of Silicon Compound, Part 2, pp. 1291-1357), *Deschler & al.* (Angew. Chem., Int. Ed. Engl., 25, 1986, 236), et dans la demande de brevet JP 9 100 353.

Lorsque la silicone aminée est un dérivé de silice, il s'agit avantageusement selon l'invention d'un support de silice, sous forme de particules, greffées par au moins un radical hydrocarboné azoté tel que défini ci-dessus. De manière préférentielle, les particules de silice sont des nanoparticules d'un diamètre moyen compris entre 10 et 100 nm, de préférence compris entre 30 et 60 nm, présentant une surface spécifique d'environ 90 à 100 m²/g. Le support de silice peut être une couche de silice qui enrobe un support métallique, il s'agit avantageusement selon l'invention d'un support de tungstène ou d'or. Le support est avantageusement une particule de diamètre allant de 0.5 à 2 µm environ, de préférence 1 µm environ.

Le diamètre moyen des particules est défini en taille et mesuré par diffraction de lumière ou par visualisation au microscope électronique à transmission. La mesure de surface spécifique est effectuée par isotherme d'absorption B.E.T.

Les particules selon l'invention sont de préférence préparées selon la méthode dite de « Stöber », par condensation de tétraéthoxysilane en milieu alcoolique aqueux ammoniacal, suivie d'une distillation à volume constant de l'ammoniac et de l'alcool. L'eau dans laquelle des particules de silice ont été transférées est retirée par distillation azéotropique en présence de toluène, suivie d'un chauffage sous vide permettant de retirer les couches d'hydratation en surface de la silice.

Ce type de préparation permet d'obtenir des particules bien qualibrées (faible dispersité des diamètres), dépourvues de macro ou micropores (les opérations d'accrochage ont lieu exclusivement en surface) et dépourvues de contaminations (en particulier absence des sels qu'on trouve dans la silice de précipitation).

Les particules de silices ou les couches de silice enrobant un support métallique sont ensuite fonctionalisées par greffage de silanes comprenant au moins un radical hydrocarboné azoté décrit ci-dessus, en particulier de manière covalente.

De nombreux modes opératoires décrivent l'utilisation d'alkoxysilanes ou de chlorosilanes pour fonctionnaliser des surfaces de silice soit sous forme d'un mélange préhydrolysé, soit, si la réaction n'a pas lieu en milieu au moins partiellement aqueux, en réagissant avec l'eau des couches d'hydratation de surface.

Une autre voie de synthèse décrite dans la littérature (Shoji Hara, Akira Dabashi Journal of Chromatography, 186 (1979) 543-552) consiste à faire réagir en milieu rigoureusement anhydre un alkoxysilane avec les siloxanes de surface des nanoparticules préalablement dépourvues de toute eau adsorbée. La réaction est effectuée pendant 12h dans du toluène à 100°C et l'alcool formé (l'éthanol) est retiré par distillation azéotropique. La silice est filtrée et rincée par différents solvants pour en retirer les siloxanes en excès qui ne s'y sont pas fixés.

De manière préférentielle, la fonctionalisation est effectuée selon ce deuxième type de préparation. La fonctionalisation de la silice peut être réalisée par différents types d'alkoxysilanes, de préférence par un silane de formule ci-après : 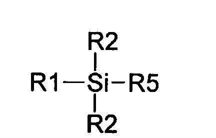 dans laquelle, R1, R2 sont définis ci-dessus et R5 représente un radical alkoxy.

De manière préférentielle, R5 représente un radical méthoxy, éthoxy, isopropyloxy, chlorure ou acétoxy.

Cette fonctionalisation peut être réalisée en présence d'autres silanes pour lesquelles R1 et/ou R2 et/ou R5 sont différents de manière à faire varier la teneur en radicaux hydrocarbonés azotés des particules obtenues. Dans ce cas, si nSi-OH est le nombre de moles de silanols de surface, et ni les nombres de moles respectifs des différentes fonctionalités i ajoutées. La réaction s'effectue de telle façon que :

nSi-OH << Sni

Ce mode de préparation permet le contrôle de la quantité relative de chaque fonction en surface des nanoparticules de silice tel que pour ki la constante de vitesse de condensation du composé i, le pourcentage molaire final d'un composé 1 en surface des nanoparticules sera voisin de klnl/Skini.

De manière avantageuse, le rapport molaire (atomes d'azote du composé siliconé azoté)/(fonctions phosphates du fragment d'acide nucléique) est compris entre 0,1 et 6000, de préférence compris entre 1 et 1000.

Le véhicule approprié est un véhicule aqueux, de préférence une solution aqueuse, usuel comme milieu de compactage des fragments d'acides nucléiques. Il peut également comprendre des additifs tels que tampons ou sels, à la condition qu'ils ne viennent pas dégrader les fragments d'acides nucléiques et/ou détruire les agrégats obtenus.

Il a été constaté que les compositions selon l'invention présentaient une stabilité améliorée des fragments d'ADN, notamment en améliorant la protection des dits fragments contre les dégradations enzymatiques, notamment par les DNAses.

La présente invention concerne également l'utilisation des composés siliconés (silicones organiques ou dérivés de silice) tels que définis ci-dessus, pour le compactage et la protection des fragments d'acides nucléiques, en particulier pour la protection contre les dégradations enzymatiques, plus spécialement par les DNAses.

La transformation de cellules peut être réalisée par toute méthode connue de l'état de la technique (notamment Jean-Yves LEGENDRE & coll., *medecine/sciences,* 1996 ; 12 ; 1334-41).

Les cellules transformées par le procédé selon l'invention peuvent être des cellules animales, végétales, bactériennes, de champignons ou de levures.

Pour les cellules animales, il peut s'agir de toute cellules d'origine animale, en particulier de mammifères, notamment d'origine humaine, mais également de cellules d'insectes.

Pour les cellules végétales, il s'agira avantageusement de cellules de plantes, monocotylédones ou dicotylédones, et plus particulièrement de plantes de culture, comme destinées ou non à l'alimentation animale ou humaine, comme le maïs, le blé, le colza, le soja, le riz, la canne à sucre, la betterave, le tabac, le coton, etc. Les cellules de plantes transformées peuvent être indifférenciées ou différenciées, comme par exemple des cellules de protoplastes ou des cellules de pollens. Dans ce dernier cas, les pollens étant riches en en DNAses, la transformation sera facilitée par la protection des fragments d'acides nucléiques au moyen des composés siliconés selon l'invention.

Pour la transformation des cellules végétales, une série de méthodes consiste à bombarder des cellules, des protoplastes ou des tissus avec des particules auxquelles sont accrochées les séquences d'ADN. Une autre série de méthodes consiste à utiliser comme moyen de transfert dans la plante un gène chimère inséré dans un plasmide Ti d'Agrobacterium tumefaciens ou Ri d'Agrobacterium rhizogenes.

D'autres méthodes peuvent être utilisées telles que la micro-injection ou l'électroporation, ou encore la précipitation directe au moyen de PEG.

L'homme du métier fera le choix de la méthode appropriée en fonction de la nature de l'organisme hôte, en particulier de la cellule végétale ou de la plante.

La présente invention a encore pour objet les plantes contenant des cellules végétales transformées ci-dessus, en particulier les plantes régénérées à partir des cellules transformées. La régénération est obtenue par tout procédé approprié qui dépende de la nature de l'espèce.

Pour les procédés de transformation des cellules végétales et de régénération des plantes, on citera notamment les brevets et demandes de brevet suivants: US 4,459,355, US 4,536,475, US 5,464,763, US 5,177,010, US 5,187,073, EP 267,159, EP 604 662, EP 672 752, US 4,945,050, US 5,036,006, US 5,100,792, US 5,371,014, US 5,478,744, US 5,179,022, US 5,565,346, US 5,484,956, US 5,508,468, US 5,538,877, US 5,554,798, US 5,489,520, US 5,510,318, US 5,204,253, US 5,405,765, EP 442 174, EP 486 233, EP 486 234, EP 539 563, EP 674 725, WO 91/02071 et WO 95/06128.

D'autres caractéristiques de l'invention apparaîtront à la lecture des exemples ci-dessous.

### Exemple I : Préparation des particules de silice fonctionnalisées.

Des nanoparticules ont été fonctionnalisées par un mélange de :
3-aminopropyldiméthyléthoxysilane 10%
triméthyléthoxysiloxane 90%
ou
octyltriméthoxysilane 1%
3-aminopropyltriméthoxysilane 1% méthyltriméthoxysilane 92%.

Dans cet exemple, les mélanges sont réalisés autant que possible avec le même type de fonctions alkoxysilanes réactives (uniquement des monométhoxy, ou des triéthoxy etc) pour minimiser les différences de réactivité.

### Mode opératoire :

Les nanoparticules une fois séchées sont manipulées dans de la verrerie flambée, sous atmosphère inerte d'argon. Tous les solvants organiques utilisés sont distillés en continus ou séchés selon les méthodes usuelles.

Dans un bicol pourvu d'un septum, d'un réfrigérant et sous pression d'argon, les nanoparticules de silice sont suspendues au moyen d'ultrasons dans du toluène. L'alkoxysilane est ajouté à la seringue en excès (2,5 10-3 mol/g SiO2 sèche).

Pour les fonctionalisations avec des mélanges d'alkoxysilanes, ces derniers sont préalablement mélangés dans un ballon, sous argon, contenant du toluène, l'ensemble est canulé sur la suspension de nanoparticules.

Le milieu réactionnel est homogénéisé aux ultrasons pendant une à deux heures puis porté au reflux du toluène pendant un minimum de trois heures pour les composés portant trois fonctions alkoxysilanes par atome de silicium ou environ 12 heures pour les composés ne comportant qu'une fonction alkoxysilane par atome de silicium.

Le milieu est ensuite centrifugé à froid (4800 tr/m, 3838 G, 4°C). Le surnageant est éliminé, le culot est resuspendu dans du toluène (anhydre) puis centrifugé. L'opération est répétée trois fois. Le culot final est mis à sécher sous vide. (aspect : poudre blanche).

Les nanoparticules ainsi obtenues peuvent être suspendues dans le solvant approprié (y compris l'eau) pour l'utilisation souhaitée ou les étapes de fonctionalisations ultérieures.

### Exemple II : Compaction et protection de l'ADN :

### Exemple II.1 : Compaction et protection avec les nanoparticules fonctionnalisées selon l'invention

Les nanoparticules fonctionalisées au moins partiellement par des 3-aminopropylsilanes sont suspendues à l'aide d'ultrasons dans de l'eau (filtrée sur membrane de filtration stérilisante) ou dans un tampon (Hépès, NaCl, P.B.S.) ne comportant pas d'amines. L'échantillon de nanoparticules est additionné sur une solution d'ADN.

Si l'ADN est additionné sur la suspension de nanoparticules, on observe une floculation due à l'agrégation de plusieurs nanoparticules autour d'un même brin d'ADN. De telles préparations permettent une protection de l'ADN contre les dégradations dues aux DNAses améliorée par rapport à l'état de la technique.

### Exemple II.2 : Compaction avec des polyaminosiloxanes

Produits utilisés :
oligomères de 3-aminopropylsilanetriols en solution aqueuse à 40 %
oligomères de N(2-aminoéthyl)-3-aminopropylméthylsilane (huile)
produits résultant de la polymérisation en présence de traces d'eau du 3-aminopropyltriméthoxysilane dans le toluène.

Pour tester l'absence de compaction en présence des monomères :
3-aminopropyltriméthoxysilane (huile)
3-aminopropylméthyldiéthoxysilane (huile)

ADN:

Divers plasmides d'origine bactérienne, supercoiled, de longueurs variables (de 10,2 kB à 3,2 kB) et de différents types (Bluscript, Lac-Z, codant pour la luciférase, la b-galaclosidase).

### 2.1 Préparations :

Les complexes ADN-Polyaminosiloxanes ont été préparés dans divers milieux : eau (filtrée sur membrane de filtration stérilisante), milieu NaCI 75mM à 150mM, milieu tampon Hépès et milieu CaCl₂.

Les mélanges ont été réalisés comme suit :

La solution aqueuse de polyaminosiloxanes concentrée est homogénéisée aux ultrasons. Un aliquot de cette solution est dilué dans la moitié du volume final en tampon et homogénéisé. L'ADN, en solution dans le tampon est ajouté volume à volume sur la solution de polyaminosiloxanes. L'ensemble est agité au moyen d'un vortex. L'ordre d'addition des différents composants peut être modifié.

La même procédure a été effectuée pour contrôle avec les monomères qui n'ont donné lieu à aucune compaction.

### 2.2 Effet de compaction, protection de l'ADN :

Lors de l'observation par microscopie électronique de mélanges entre de l'ADN et chacun des produits, on observe différentes figures d'ADN compacté dont certaines sont semblables à celles obtenues en présence d'histones ou de compactants connus comme la spermine ou les polyéthylèneimines (cf par exemple M. Garcia Ramirez, J. A. Subirana, Biopolymers, vol 34, p 285-292, 1984).

Les figures observées sont de nature différente suivant la concentration et le produit utilisé.

Une étude avec les oligomères de 3-aminopropylsilanetriols à montré nettement un effet dose : pour des rapports (mole de fonction amine du polysiloxane)/(mole de phosphate de l'ADN) croissant (de 0,1 à 6000), on observe une compaction croissante.

La technique d'exclusion du bromure d'éthydium (BET) a permis de prouver la forte capacité des produits à compacter l'ADN.

Cette compaction permet de protéger l'ADN de dégradations.

### Exemple III: transfection

Des essais de transfection ont été réalisés à différentes conditions et sur trois types de lignées cellulaires (NIH3T3, HeLa et HepG2). On observe des activités de transfection.

Exemple de préparations :
ADN à 10µg/ml, oligomères de 3-aminopropylsilanetriols à 9,1 10-7 mol/ml soit (30 moles d'amines par moles de phosphate)

ADN à 10µg/ml, oligomères de N(2-aminoéthyl)-3-aminopropylméthylsilane à 3 10-8 mol/ml (soit un équivalent amine primaire par phosphate), 9.1 10-7 mol/ml (30 équivalents amines primaires par phosphate) ou 3 10-5 mol/l (1000 équivalents amines primaires par phosphate).

Ces préparations ont donné une expression supérieure aux agents agrégeants standard polyaminés de l'état de la technique utilisés à 6 10⁻⁸ mol/ml.

### Exemple IV : transformation de plantes

Avant les essais in vivo, une évaluation de la toxicité est réalisée. Soixante à soixante dix mg de cellules sont transférées dans des fioles de 150 ml contenant 10 ml de milieu de culture et différentes concentrations d'oligomères de N(2-aminoéthyl)-3-aminopropylméthylsilane (0, 1, 2, 4 et 8 mg/l). Les cellules sont prélevées après 7 jours de culture et le poids frais des cellules est estimé. On observe une augmentation du pH (8,1) du milieu de culture pour des concentrations d'oligomères de N(2-aminoéthyl)-3-aminopropylméthylsilane de 8 mg/l et une inhibition de la croissance cellulaire, mais pas de toxicité. La molarité du N(2-aminoéthyl)-3-aminopropylméthylsilane pour le plus grand rapport amine primaire par phosphate (5333) est de 0,16 mM et est équivalent à la molarité du N(2-aminoéthyl)-3-aminopropylméthylsilane dans le test de toxicité à 2 mg/l. Ces résultats montrent que les concentrations utilisées pour le bombardement ne sont pas phytotoxiques.

Des essais de transformation de plantes ont été réalisés sur des cellules d'arabidopsis (souche T 87) obtenus selon la méthode décrite par Axelos ( Axelos M. et al., 1992 *Plant Physiol.* 30 (1), 123-128). La technique utilisée est le bombardement (Vain Ph et al., 1993 Plant *Cell, Tissue and Organ Culture.* 33 237-246). On observe une expression transitoire ainsi qu'une expression stable (obtention de cals), équivalentes à celles obtenues avec la spermidine.

Exemples de préparations ou bombardement :
- Des particules de tungstène (Sylvania Chemicals) M17, de diamètre moyen de 1 µm, sont stérilisées dans de l'éthanol à 95°C GL à raison de 1g/20ml. Cinq cent µl de particules en suspension sont prélevés, lavés 3 fois dans de l'eau milli Q stérile et repris dans 500 µl d'eau final. La précipitation de l'ADN autour des particules est réalisée selon le protocole ci-après. On réalise le mélange suivant en ajoutant dans l'ordre :
   50 µl de particules M17, 10 µg d'ADN, 50 µl de CaCl2 2,5M, plus N(2-aminoéthyl)-3-aminopropylméthylsilane de façon à obtenir les rapports amine primaire par phosphate de 167 ; 667 ; 1333 ; 2667 et 5333. Après 10 minutes, le mélange ainsi précipité est utilisé pour réaliser 2 tirs.
- 10µg d'ADN sont mélangés à une solution de 2,5 mg de particules de tungstène siliconée avec du 3-aminopropyltriméthoxysilane dans 50 µl d'eau. Après 10 minutes, le mélange ainsi précipité est utilisé pour réaliser 2 tirs. On observe une expression transitoire sensiblement équivalente à celle du complexe précédent.

## Revendications

1. Procédé de transformation de cellules par lequel on introduit dans les cellules une quantité appropriée de fragments d'acides nucléiques, caractérisé en ce que les fragments d'acides nucléiques sont introduits sous la forme d'une composition d'acide nucléique qui comprend dans un véhicule approprié au moins un fragment d'acide nucléique et au moins un agent compactant comprenant une silicone azotée.

2. Procédé selon la revendication 1, caractérisé en ce que le fragment d'acide nucléique est une séquence nucléotidique pouvant être de type ADN ou ARN, de préférence de type ADN.

3. Procédé selon l'une des revendications 1 ou 2, caractérisé en ce que la silicone azotée est un composé comprenant dans sa structure au moins un atome de silicium sur lequel est greffé au moins un radical hydrocarboné azoté.

4. Procédé selon la revendication 3, caractérisé en ce que la structure de la silicone azotée est un oligomère.

5. Procédé selon la revendication 3, caractérisé en ce que l'atome d'azote dans le radical hydrocarboné se trouve sous la forme d'une amine primaire, secondaire ou tertiaire, de préférence primaire, éventuellement sous la forme d'un sel d'ammonium, ou encore sous la forme de fonctions de type guanidine ou amidine.

6. Procédé selon la revendication 3, caractérisé en ce que le radical hydrocarboné azoté est un radical hydrocarboné aminé, comprenant au moins une fonction amine, de préférence au moins une fonction amine primaire (-NH2).

7. Procédé selon la revendication 6, caractérisé en ce que le radical hydrocarboné aminé est choisi parmi les radicaux 3-aminopropyle ou 3-(2-aminoéthylamino)-propyle.

8. Procédé selon l'une des revendications 1 à 7, caractérisé en ce que les silicones azotées sont choisies parmi les silicones organiques ou les dérivés de silice.

9. Procédé selon la revendication 8, caractérisé en ce que les silicones organiques comprennent au moins un motif silicone suivant :
Si R1 O_{(3-x-y)/2} R2ₓ R3_{y}
dans lequel,
R1 est un radical hydrocarboné azoté,
R2 représente le radical R1, un radical hydroxyle ou un radical hydrocarboné,
R3 représente R2 ou le radical de formule 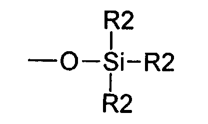
dans laquelle R2 est défini ci-dessus,
x et y, identiques ou différents sont 0, 1 ou 2, étant entendu que la somme x+y est inférieure ou égale à 3.

10. Procédé selon la revendication 8, caractérisé en ce que la silicone azotée est choisie parmi les silicones de formule générale I ou II suivantes : 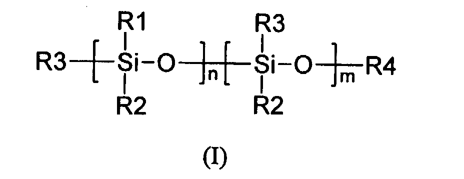 ou 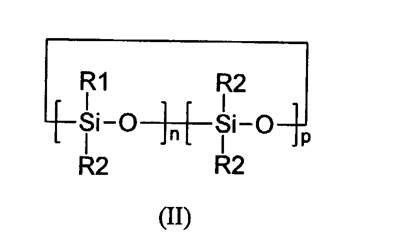 dans lesquelles,
R1, R2 et R3 sont définis dans la revendication 8,
R4 représente un atome d'hydrogène, un radical hydrocarboné ou un radical de formule 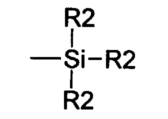
dans laquelle R2 est défini précédemment,
n est au moins égal à 1, m est supérieur ou égal à 0 et p est supérieur ou égal à 0, à la condition que la somme n+p soit supérieure ou égale à 3.

11. Procédé selon la revendication 10, caractérisé en ce que n est compris entre 1 et 10, plus préférentiellement entre 5 et 9, et m ou p sont compris entre 0 et 9.

12. Procédé selon la revendication 10, caractérisé en ce que dans les silicones de formule générale I, R2 représente un radical hydroxyle ou un radical hydrocarboné, en particulier alkyle, R3 représente un radical hydroxyle et R4 représente un atome d'hydrogène, n est compris entre 7 et 9 et m est égal à 0.

13. Procédé selon la revendication 8, caractérisé en ce que la silicone azotée dérivé de silice est constituée par un support de silice greffées par au moins un radical hydrocarboné azoté.

14. Procédé selon la revendication 13, caractérisé en ce que le support est une particule.

15. Procédé selon la revendication 14, caractérisé en ce que les particules de silice sont des nanoparticules d'un diamètre moyen compris entre 10 et 100 nm, de préférence compris entre 30 et 60 nm.

16. Procédé selon la revendication 15, caractérisé en ce que les particules présentent une surface spécifique d'environ 90 à 100 m²/g.

17. Procédé selon la revendication 14, caractérisé en ce que le support est une couche de silice enrobant un support métallique.

18. Procédé selon la revendication 17, caractérisé en ce que le support est une particule de diamètre allant de 0.5 à 2 µm environ, de préférence 1 µm environ.

19. Procédé selon l'une des revendications 1 à 18, caractérisé en ce que le rapport molaire (atomes d'azote du composé siliconé azoté)/(fonctions phosphates du fragment d'acide nucléique) est compris entre 0,1 et 6000, de préférence compris entre 1 et 1000.

20. Utilisation des composés siliconés azotés tels que définis dans les revendications 3 à 19, pour le compactage de fragments d'acides nucléiques.

21. Utilisation des dérivés siliconés azotés tels que définis dans les revendications 3 à 19 pour protéger les fragments d'acides nucléiques contre les dégradations enzymatiques, en particulier par les DNAses.

22. Procédé selon la revendication 1, caractérisé en ce que les cellules transformées sont choisies parmi les cellules animales, végétales, bactériennes, de champignons ou de levures.

23. Procédé selon la revendication 22, caractérisé en ce que les cellules végétales sont choisies parmi les cellules de plantes, monocotylédones ou dicotylédones, et plus particulièrement de plantes de culture, comme destinées ou non à l'alimentation animale ou humaine, comme le maïs, le blé, le colza, le soja, le riz, la canne à sucre, la betterave, le tabac, le coton.

24. Procédé selon l'une des revendications 22 à 23, caractérisé en ce que des plantes sont régénérées à partir des cellules végétales transformées.
